# EUROPEAN PATENT APPLICATION

(11) **EP 4 494 542 A1**
(43) Date of publication of application: **22.01.2025**
(21) Application number: 23770516.5
(22) Date of filing: 06.03.2023
(51) Int. Cl.: A61B 1/00, A61B 1/06, A61B 1/07

(54) **MEDICAL LIGHT SOURCE DEVICE AND MEDICAL OBSERVATION SYSTEM**

(30) Priority: 16.03.2022 JP 2022041925
(71) Applicant: Sony Olympus Medical Solutions Inc., Hachioji-shi, Tokyo 192-0904 (JP)
(72) Inventor: KITAMURA, Ken, Hachioji-shi, Tokyo 192-0904 (JP)
(74) Representative: D Young & Co LLP
(86) International application number: PCT/JP2023/008407
(87) International publication number: WO 2023/176560

(57) **Abstract**

A medical light source device 3 includes a visible light source 31 that emits normal light in a visible wavelength band, and a plurality of excitation light sources 32 to 34 that emits a plurality of kinds of excitation light corresponding to a plurality of kinds of drugs each emitting fluorescence upon irradiation with the excitation light.

## Description

### Field

The present disclosure relates to a medical light source device and a medical observation system.

### Background

In conventional medical observation systems that observe subjects, there is known a system that irradiates a drug administered into a subject with excitation light to excite the drug, observing fluorescence emitted from the drug (e.g., see Patent Literature 1).

### Citation List

### Patent Literature

Patent Literature 1: JP 2018-42676 A

### Summary

### Technical Problem

However, the medical observation system described in Patent Literature 1 is configured for only one type of drug such as indocyanine green, and fluorescent observation corresponding to a plurality of kinds of drugs is not available. Therefore, convenience cannot be improved.

The present disclosure has been made in view of the above, and an object thereof is to provide a medical light source device and a medical observation system that are configured to perform fluorescent observation corresponding to the plurality of kinds of drugs with improved convenience.

### Solution to Problem

To solve the above-described problem and achieve the object, a medical light source device according to the present disclosure includes: a visible light source configured to emit normal light in a visible wavelength band; and a plurality of excitation light sources configured to emit a plurality of kinds of excitation light corresponding to a plurality of kinds of drugs each emitting fluorescence upon irradiation with the excitation light.

A medical observation system according to the present disclosure includes: a medical light source device including a visible light source configured to emit normal light in a visible wavelength band, and a plurality of excitation light sources configured to emit a plurality of kinds of excitation light corresponding to a plurality of kinds of drugs each emitting fluorescence upon irradiation with the excitation light; an imaging device configured to image the normal light emitted from the visible light source and reflected by a subject and fluorescence emitted from the plurality of kinds of drugs in the subject upon emission of the plurality of kinds of excitation light from the plurality of excitation mirror light sources; a display device configured to display a captured image captured by the imaging device; and a control device configured to control the medical light source device, the imaging device and the display device.

### Advantageous Effects of Invention

According to the medical light source device and the medical observation system according to the present disclosure, fluorescent observation corresponding to the plurality of kinds of drugs can be performed, with improved convenience.

### Brief Description of Drawings

FIG. 1 is a diagram illustrating a schematic configuration of a medical observation system according to a first embodiment.
FIG. 2 is a block diagram illustrating a configuration of a light source device.
FIG. 3 is a block diagram illustrating configurations of a camera head and a control device.
FIG. 4 is a diagram illustrating a configuration of an imaging unit.
FIG. 5 is a graph illustrating a sensitivity of a first imaging element.
FIG. 6 is a graph illustrating a sensitivity of a second imaging element.
FIG. 7 is a graph illustrating a sensitivity of a third imaging element.
FIG. 8 is a diagram illustrating operations of the control device.
FIG. 9 is a diagram illustrating a first modification of the first embodiment.
FIG. 10 is a diagram illustrating operations of a control device according to a second embodiment.
FIG. 11 is a diagram illustrating a second modification of the second embodiment.
FIG. 12 is a diagram illustrating a third modification of the first and second embodiments.
FIG. 13 is a graph illustrating the third modification of the first and second embodiments.
FIG. 14 is a graph illustrating the third modification of the first and second embodiments.
FIG. 15 is a graph illustrating the third modification of the first and second embodiments.

### Description of Embodiments

Modes for carrying out the present disclosure (hereinafter referred to as embodiments) will be described below with reference to the drawings. Note that the present disclosure is not limited to the embodiments described below. Furthermore, in illustration of the drawings, the same portions are denoted by the same reference numerals.

### (First embodiment)

### [Schematic configuration of medical observation system]

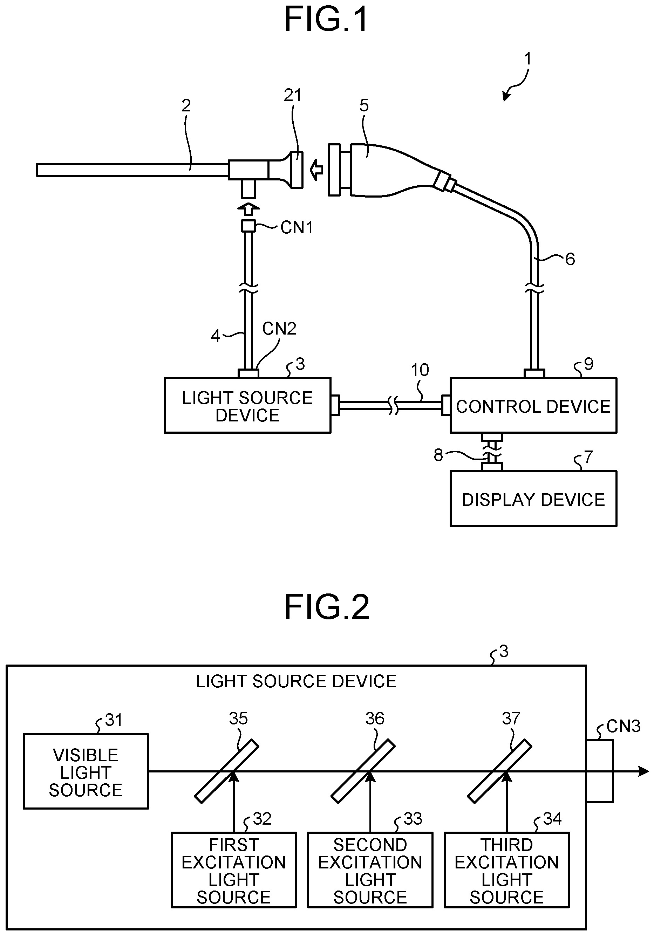

FIG. 1 is a diagram illustrating a schematic configuration of a medical observation system 1 according to a first embodiment.

The medical observation system 1 is a system that is used in a medical field to observe a subject (in vivo). As illustrated in FIG. 1, the medical observation system 1 includes an insertion section 2, a light source device 3, a light guide 4, a camera head 5, a first transmission cable 6, a display device 7, a second transmission cable 8, a control device 9, and a third transmission cable 10.

In the present first embodiment, the insertion section 2 includes a rigid endoscope. In other words, the insertion section 2 has an elongated shape that is entirely rigid or has a part soft and the other part rigid, for insertion into a living body. The insertion section 2 is internally provided with an optical system (not illustrated) that is constituted by using one or a plurality of lenses to collect light to form a subject image.

The light source device 3 corresponds to a medical light source device according to the present disclosure. To this light source device 3, a connector CN2 of the light guide 4 is connected to supply light (normal light such as white light, or excitation light) specified by the control device 9 to an incident end of the light guide 4 under the control of the control device 9. In the present first embodiment, the light source device 3 is constituted separately from the control device 9, but is not limited to this configuration, and may be provided in the control device 9.

Note that a detailed configuration of the light source device 3 will be described in "Configuration of light source device" which is described later.

The light guide 4 includes a connector CN1 that is provided on an emission end side to be detachably connected to the insertion section 2, and the connector CN2 that is provided on the incident end side to be detachably connected to a connector CN3 (see FIG. 2) of the light source device 3. The light guide 4 supplies light (normal light such as white light, or excitation light) supplied from the light source device 3 to the insertion section 2. The light supplied to the insertion section 2 is emitted into the living body from a distal end of the insertion section 2, and normal light or excitation light reflected in the living body and fluorescence emitted from a fluorescent substance (drug) in the living body excited by the excitation light are collected by the optical system in the insertion section 2.

The camera head 5 corresponds to an imaging device according to the present disclosure. The camera head 5 is detachably connected to an eye piece 21 of the insertion section 2. Then, under the control of the control device 9, the camera head 5 captures the subject image formed by collecting light by the insertion section 2 and generates an image signal (hereinafter, described as a captured image).

Note that a detailed configuration of the camera head 5 will be described in "Configuration of camera head" which is described later.

The first transmission cable 6 has one end that is detachably connected to the control device 9, and the other end that is detachably connected to the camera head 5. Then, the first transmission cable 6 transmits the captured image and the like output from the camera head 5 to the control device 9, and transmits a control signal, a synchronization signal, clock, power, and the like output from the control device 9 to the camera head 5.

Note that, in transmission of the captured image and the like from the camera head 5 to the control device 9 via the first transmission cable 6, the captured image and the like may be transmitted using an optical signal or may be transmitted using an electric signal. The same applies to transmission of the control signal, the synchronization signal, and the clock from the control device 9 to the camera head 5 via the first transmission cable 6.

The display device 7 includes a display using liquid crystal, organic electro luminescence (EL), or the like, and displays an image based on a video signal from the control device 9 under the control of the control device 9.

The second transmission cable 8 has one end that is detachably connected to the display device 7, and the other end that is detachably connected to the control device 9. Then, the second transmission cable 8 transmits the video signal processed by the control device 9 to the display device 7.

The control device 9 includes a central processing unit (CPU), a field-programmable gate array (FPGA), or the like to integrally control operations of the light source device 3, the camera head 5, and the display device 7.

Note that a detailed configuration of the control device 9 will be described in "Configuration of control device" which is described later.

The third transmission cable 10 has one end that is detachably connected to the light source device 3, and the other end that is detachably connected to the control device 9. The third transmission cable 10 transmits the control signal transmitted from the control device 9 to the light source device 3.

### [Configuration of light source device]

FIG. 2 is a block diagram illustrating a configuration of the light source device 3.

Next, the configuration of the light source device 3 will be described with reference to FIG. 2.

As illustrated in FIG. 2, the light source device 3 includes a visible light source 31, first to third excitation light sources 32 to 34, and first to third dichroic mirrors 35 to 37.

The visible light source 31 outputs (emits) normal light such as white light in a visible wavelength band. In the present first embodiment, the visible light source 31 includes a light emitting diode (LED) that emits the white light (normal light).

The first excitation light source 32 corresponds to an excitation light source according to the present disclosure. The first excitation light source 32 includes a semiconductor laser that emits excitation light (hereinafter, described as blue excitation light) having a peak wavelength in a blue wavelength band. The blue excitation light excites at least one type of fluorescent substance (drug) to cause the fluorescent substance to generate fluorescence. Hereinafter, it is assumed that a peak wavelength of the fluorescence emitted from the fluorescent substance when the blue excitation light excites the fluorescent substance is different from the peak wavelength of the blue excitation light, but is included in the blue wavelength band. In addition, the fluorescence is described as blue fluorescence.

The second excitation light source 33 corresponds to an excitation light source according to the present disclosure. The second excitation light source 33 includes a semiconductor laser that emits excitation light (hereinafter, described as red excitation light) having a peak wavelength in a red wavelength band. The red excitation light excites at least one type of fluorescent substance (drug) different from the fluorescent substance excited by the blue excitation light described above to cause the fluorescent substance to generate fluorescence. Hereinafter, it is assumed that a peak wavelength of the fluorescence emitted from the fluorescent substance when the red excitation light excites the fluorescent substance is different from the peak wavelength of the red excitation light but is included in the red wavelength band. In addition, the fluorescence is described as red fluorescence.

The third excitation light source 34 corresponds to an excitation light source according to the present disclosure. The third excitation light source 34 includes a semiconductor laser that emits excitation light (hereinafter, described as infrared excitation light) having a peak wavelength in an infrared wavelength band. The infrared excitation light excites at least one type of fluorescent substance (drug) different from the above fluorescent substances excited by the blue excitation light and the red excitation light to cause the fluorescent substance to generate fluorescence. Hereinafter, it is assumed that a peak wavelength of fluorescence emitted from the fluorescent substance when the infrared excitation light excites the fluorescent substance is different from the peak wavelength of the infrared excitation light, but is included in the infrared wavelength band. In addition, the fluorescence is described as infrared fluorescence.

The visible light source 31 and first to third excitation light sources 32 to 34 which are described above are arranged at the following positions relative to the connector CN3 being an emission port at which the visible light, the blue excitation light, the red excitation light, and the infrared excitation light are emitted from the light source device 3.

As illustrated in FIG. 2, the visible light source 31 of the visible light source 31 and the first to third excitation light sources 32 to 34 is arranged at a position farthest from the connector CN3.

An arrangement order of the first to third excitation light sources 32 to 34 relative to the connector CN3 corresponds to an order of magnitude of the peak wavelengths of the blue excitation light, the red excitation light, and the infrared excitation light.

Specifically, of the first to third excitation light sources 32 to 34, the first excitation light source 32 having the smallest peak wavelength of the excitation light to be emitted is arranged at a position farthest from the connector CN3. Meanwhile, of the first to third excitation light sources 32 to 34, the third excitation light source 34 having the largest peak wavelength of the excitation light to be emitted is arranged at a position closest to the connector CN3. The second excitation light source 33 is arranged at a position between the first and third excitation light sources 32 and 34.

In other words, in the present first embodiment, the larger peak wavelengths the emitted excitation light of the first to third excitation light sources 32 to 34 have, the closer the first to third excitation light sources 32 to 34 are arranged to the connector CN3. Furthermore, the visible light source 31 is arranged at a position farther from the connector CN3 than the first to third excitation light sources 32 to 34.

The first dichroic mirror 35 is a dichroic mirror that transmits the normal light and reflects the blue excitation light in the same direction as a travel direction of the normal light.

The second dichroic mirror 36 is a dichroic mirror that transmits the normal light and the blue excitation light and reflects the red excitation light in the same direction as the travel directions of the normal light and the blue excitation light.

The third dichroic mirror 37 is a dichroic mirror that transmits the normal light, the blue excitation light, and the red excitation light and reflects the infrared excitation light in the same direction as the travel directions of the normal light, the blue excitation light, and the red excitation light.

### [Configuration of camera head]

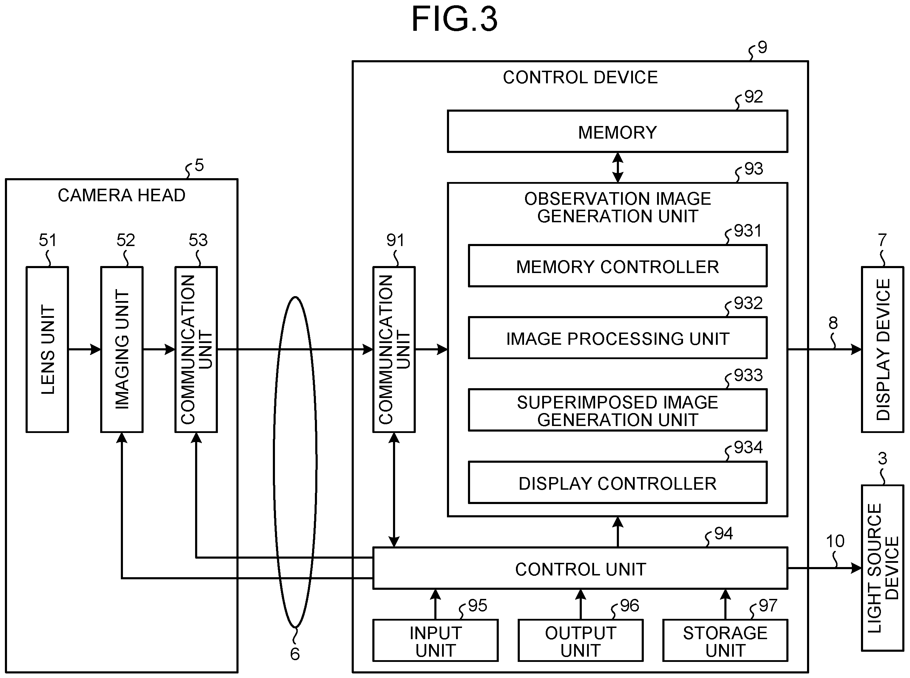

FIG. 3 is a block diagram illustrating configurations of the camera head 5 and the control device 9.

Next, the configuration of the camera head 5 will be described with reference to FIG. 3.

As illustrated in FIG. 3, the camera head 5 includes a lens unit 51, an imaging unit 52, and a communication unit 53.

The lens unit 51 is constituted by using one or a plurality of lenses and captures the subject image formed by collecting light by the insertion section 2 to form an image on an imaging surface of the imaging unit 52 (first to third imaging elements 525 to 527).

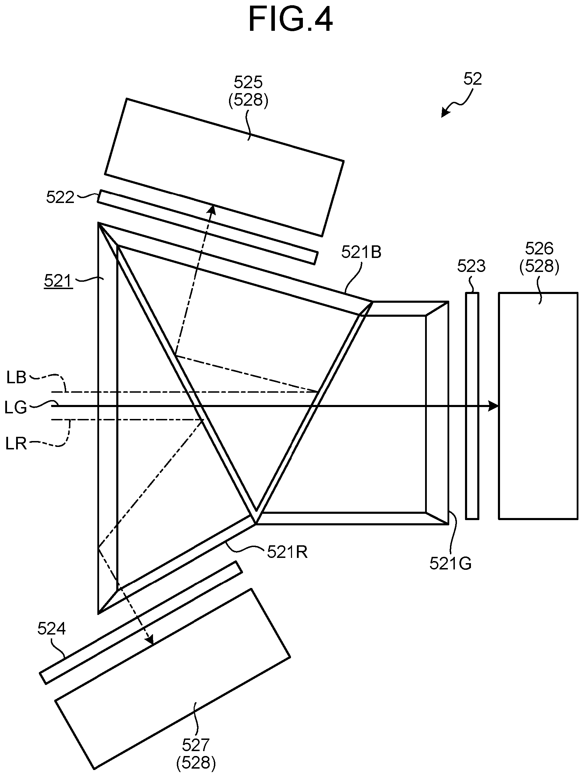

FIG. 4 is a diagram illustrating a configuration of the imaging unit 52.

The imaging unit 52 captures an image inside the living body under the control of the control device 9. As illustrated in FIG. 2, the imaging unit 52 includes a dichroic prism 521, first to third excitation light cut filters 522 to 524, and the first to third imaging elements 525 to 527.

As illustrated in FIG. 4, the dichroic prism 521 separates the subject image from the lens unit 51 into light LB in the blue wavelength band, light LG in a green wavelength band, and light LR in the red and infrared wavelength bands. Note that the blue wavelength band of the light LB includes a partial wavelength band of the normal light, the peak wavelength of the blue excitation light, and a peak wavelength of the blue fluorescence. In addition, the green wavelength band of the light LG includes a partial wavelength band of the normal light. Furthermore, the red and infrared wavelength bands of the light LR include the peak wavelength of the red excitation light, a peak wavelength of the red fluorescence, the peak wavelength of the infrared excitation light, and a peak wavelength of the infrared fluorescence.

The first excitation light cut filter 522 is arranged at a position facing a first emergent surface 521B (FIG. 4) of the dichroic prism 521 from which the light LB is emitted. Then, the first excitation light cut filter 522 removes the blue excitation light, the red excitation light, and the infrared excitation light from the incident light, and transmits the other light.

The second excitation light cut filter 523 is arranged at a position facing a second emergent surface 521G (FIG. 4) of the dichroic prism 521 from which the light LG is emitted. Then, the second excitation light cut filter 523 removes the blue excitation light, the red excitation light, and the infrared excitation light from the incident light, and transmits the other light.

The third excitation light cut filter 524 is arranged at a position facing a third emergent surface 521R (FIG. 4) of the dichroic prism 521 from which the light LR is emitted. Then, the third excitation light cut filter 524 removes the blue excitation light, the red excitation light, and the infrared excitation light from the incident light, and transmits the other light.

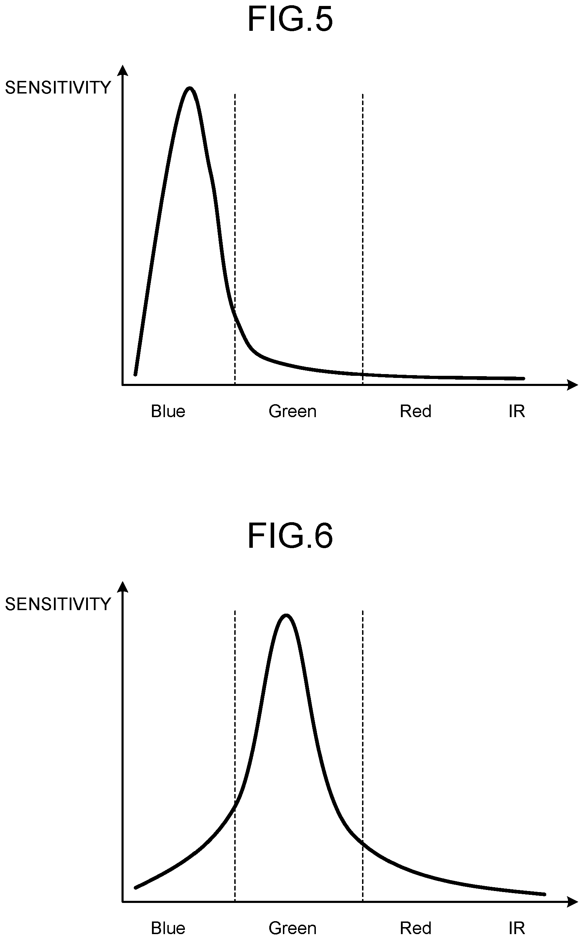

FIG. 5 is a graph illustrating a sensitivity of the first imaging element 525.

The first imaging element 525 is arranged at a position facing the first emergent surface 521B across the first excitation light cut filter 522. The first imaging element 525 includes a charge coupled device (CCD), a complementary metal oxide semiconductor (CMOS), or the like that receives, of the light LB emitted from the first emergent surface 521B, the light transmitted through the first excitation light cut filter 522, and converts the received light into an electrical signal. Furthermore, as illustrated in FIG. 5, the first imaging element 525 is configured to have a high sensitivity to light mainly in the blue wavelength band.

Note that, in the following description, a captured image obtained by imaging in the first imaging element 525 is described as a first captured image.

FIG. 6 is a graph illustrating a sensitivity of the second imaging element 526.

The second imaging element 526 is arranged at a position facing the second emergent surface 521G across the second excitation light cut filter 523. The second imaging element 526 includes CCD, CMOS, or the like that receives, of the light LG emitted from the second emergent surface 521G, the light transmitted through the second excitation light cut filter 523, and converts the received light into an electrical signal. Furthermore, as illustrated in FIG. 6, the second imaging element 526 is configured to have a high sensitivity to light mainly in the green wavelength band.

Note that, in the following description, a captured image obtained by imaging in the second imaging element 526 is described as a second captured image.

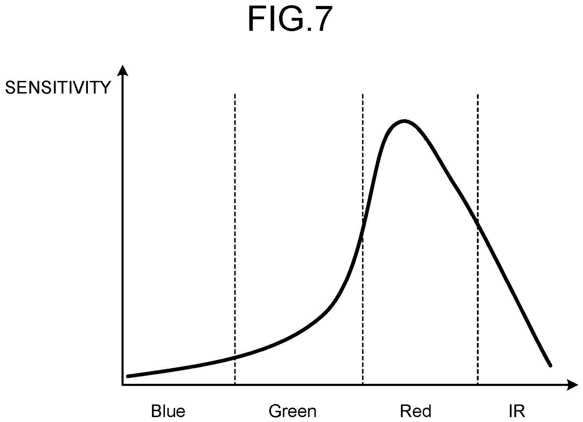

FIG. 7 is a graph illustrating a sensitivity of the third imaging element 527.

The third imaging element 527 is arranged at a position facing the third emergent surface 521R across the third excitation light cut filter 524. The third imaging element 527 includes CCD, CMOS, or the like that receives, of the light LR emitted from the third emergent surface 521R, the light transmitted through the third excitation light cut filter 524, and converts the received light into an electrical signal. Furthermore, as illustrated in FIG. 7, the third imaging element 527 is configured to have a high sensitivity to light mainly in the red and infrared wavelength bands.

In the present first embodiment, the first to third imaging elements 525 to 527 each include CMOS as a rolling shutter imaging element in which a plurality of pixels is two-dimensionally arranged in horizontal lines.

The communication unit 53 is an interface that communicates with the control device 9 via the first transmission cable 6. The communication unit 53 transmits the first to third captured images (digital signals) output from the imaging unit 52 to the control device 9.

### [Configuration of control device]

Next, the configuration of the control device 9 will be described with reference to FIG. 3.

As illustrated in FIG. 3, the control device 9 includes a communication unit 91, a memory 92, an observation image generation unit 93, a control unit 94, an input unit 95, an output unit 96, and a storage unit 97.

The communication unit 91 is an interface that communicates with the camera head 5 (communication unit 53) via the first transmission cable 6. Then, the communication unit 91 receives the first to third captured images output from the communication unit 53.

Note that an image processing unit 932 which is described later generates a captured image of one frame from the first to third captured images mainly obtained by imaging the normal light. In the following description, for convenience of description, the first to third captured images mainly obtained by imaging the normal light are collectively described as a normal-light image. Furthermore, the image processing unit 932 which is described later generates a captured image of one frame from a first to third captured images mainly obtained by imaging fluorescence such as the blue fluorescence, the red fluorescence, and the infrared fluorescence. In the following description, for convenience of description, the first to third captured images mainly obtained by imaging the fluorescence such as the blue fluorescence, the red fluorescence, and the infrared fluorescence are collectively described as a fluorescence image. Furthermore, in the following description, the first to third imaging elements 525 to 527 are collectively described as an imaging element 528 (FIG. 4).

The memory 92 includes, for example, a dynamic random access memory (DRAM) or the like. The memory 92 is configured to temporarily store a plurality of frames of the normal-light image and the fluorescence image that are sequentially output from the camera head 5 (communication unit 53) and received by the communication unit 91.

The observation image generation unit 93 processes each of the normal-light image and the fluorescence image under the control of the control unit 94. As illustrated in FIG. 3, the observation image generation unit 93 includes a memory controller 931, the image processing unit 932, a superimposed image generation unit 933, and a display controller 934.

The memory controller 931 controls writing and reading of the normal-light image and the fluorescence image to and from the memory 92. Specifically, the memory controller 931 sequentially writes the normal-light image and the fluorescence image sequentially output from the camera head 5 (communication unit 53) and received by the communication unit 91, in the memory 92. In addition, the memory controller 931 reads each of the normal-light image and the fluorescence image from the memory 92 with specific timing, and inputs each of the read normal-light image and fluorescence image to the image processing unit 932.

The image processing unit 932 performs known image processing on each of the input normal-light image and fluorescence image. Note that the image processing performed on the normal-light image and the image processing performed on the fluorescence image may be different from each other.

The superimposed image generation unit 933 performs superimposition processing of generating a superimposed image by superimposing the fluorescence image on which the image processing has been performed in the image processing unit 932, on the normal-light image on which the image processing has been performed in the image processing unit 932.

Here, as the superimposition processing, first and second superimposition processing described below can be exemplified. Note that, in the following description, a region of the fluorescence image including pixels whose brightness values are equal to or larger than a specific threshold is described as a fluorescent region (region in which the blue fluorescence, the red fluorescence, and the infrared fluorescence are detected).

The first superimposition processing is processing of replacing a region of the normal-light image at the same positions as those of the pixels of the fluorescent region with an image of the fluorescent region of the fluorescence image.

The second superimposition processing is processing (so-called alpha blending) of changing the brightness of the colors indicating the blue fluorescence, the red fluorescence, and the infrared fluorescence applied to each of the pixels in the region of the normal-light image at the same positions as those of the pixels of the fluorescent region, according to the brightness value at the position of each pixel in the fluorescent region of the fluorescence image.

The display controller 934 generates the video signal for displaying the superimposed image generated in the superimposed image generation unit 933, under the control of the control unit 94. Then, the display controller 934 outputs the video signal to the display device 7 via the second transmission cable 8. Therefore, the display device 7 displays the superimposed image based on the video signal.

The control unit 94 is implemented by executing various programs stored in the storage unit 97 by a controller such as CPU or a micro processing unit (MPU), and controls the operations of the light source device 3, the camera head 5, and the display device 7 and controls the entire operations of the control device 9. Note that the control unit 94 is not limited to the CPU or the MPU, and may include an integrated circuit such as an application specific integrated circuit (ASIC) or FPGA. Note that functions of operating the light source device 3 and the camera head 5 by the control unit 94 will be described in "Operations of control device" which is described later.

The input unit 95 is constituted by using an operation device such as a mouse, a keyboard, and a touch screen, and receives a user operation from a user such as a doctor. Then, the input unit 95 outputs an operation signal according to the user operation, to the control unit 94.

The output unit 96 is constituted by using a speaker, a printer, or the like, and outputs various information.

The storage unit 97 stores the programs executed by the control unit 94, information necessary for processing in the control unit 94, and the like.

### [Operations of control device]

Next, the operations of the above control device 9 will be described.

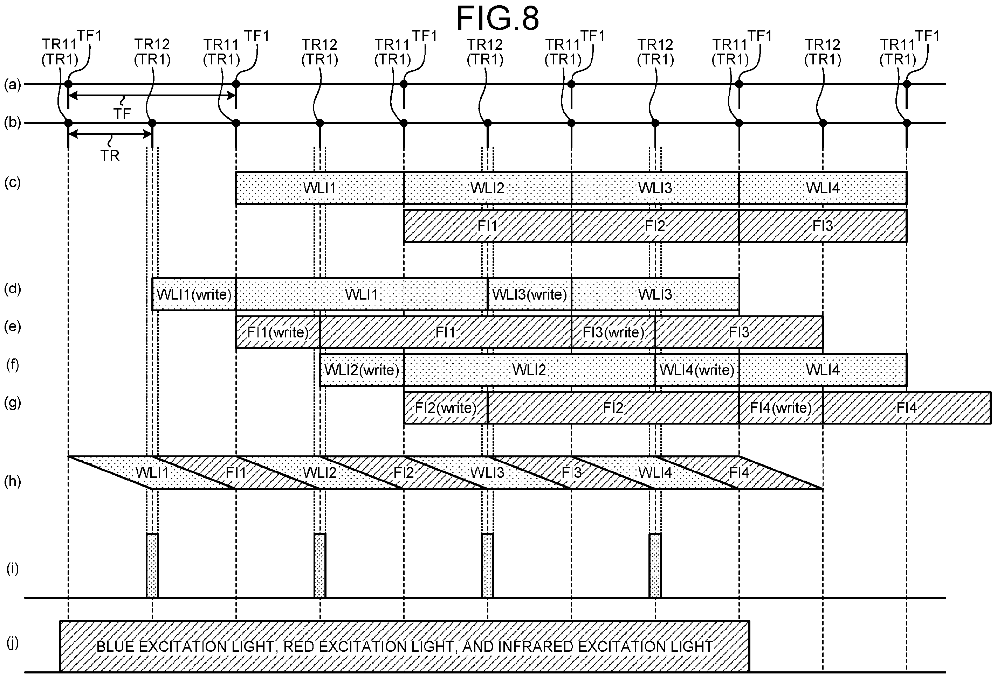

FIG. 8 is a diagram illustrating operations of the control device 9. Specifically, a diagram of (a) of FIG. 8 illustrates timing at which the control device 9 outputs the video signal according to a captured image (superimposed image) of one frame to the display device 7 to cause the display device 7 to display the captured image. Note that in (a) of FIG. 8, "TF1" indicates a display timing of the captured image. Furthermore, "TF" indicates a frame period in which the display device 7 is caused to display a captured image of one frame. A diagram of (b) of FIG. 8 illustrates timing at which the imaging element 528 is caused to capture an image. Note that in (b) of FIG. 8, "TR1" indicates an imaging timing. Furthermore, "TR" indicates an imaging period of the imaging element 528. A diagram of (c) of FIG. 8 illustrates a captured image (superimposed image) output (displayed) on the display device 7. A diagram of (d) of FIG. 8 illustrates a captured image stored in a bank 1 in the memory 92. A diagram of (e) of FIG. 8 illustrates a captured image stored in a bank 2 in the memory 92. A diagram of (f) of FIG. 8 illustrates a captured image stored in a bank 3 in the memory 92. A diagram of (g) of FIG. 8 illustrates a captured image stored in a bank 4 in the memory 92. In (h) of FIG. 8, a diagram of exposure timing of the imaging element 528 is illustrated, in which the vertical axis represents horizontal lines of the imaging element 528 (the top indicates the uppermost horizontal line (the first horizontal line), and the bottom indicates the lowermost horizontal line (the last line)), and the horizontal axis represents time. Then, a parallelogram region is a region that contributes to generation of the normal-light image or the fluorescence image. A diagram of (i) of FIG. 8 illustrates lighting timing of the visible light source 31. A diagram of (j) of FIG. 8 illustrates lighting timings of the first to third excitation light sources 32 to 34.

The control unit 94 controls the imaging element 528 as described below.

Specifically, the control unit 94 performs exposure control using so-called rolling shutter to cause the imaging element 528 to start exposure with imaging timing TR1 sequentially from the uppermost horizontal line to the lowermost horizontal line and to read sequentially from the uppermost horizontal line to the lowermost horizontal line with the next imaging timing TR1 after a predetermined period (so-called shutter speed) has elapsed from the start of the exposure.

Note that, in the present first embodiment, a drive mode of the imaging element 528 is set to a drive mode in which a readout period of reading charges accumulated in a plurality of pixels of the imaging element 528 is long (the readout period has a length the same as that of an imaging period TR illustrated in (b) of FIG. 8) and therefore an entire line exposure period is not available. Here, the entire line exposure period is a period in which all the horizontal lines in an effective pixel region in the imaging element 528 are simultaneously exposed.

In the present first embodiment, as illustrated in (a) of FIG. 8 and (b) of FIG. 8, the imaging period TR is set to a half of the frame period TF (e.g., the frame period TF: 1/60 seconds and the imaging period TR: 1/120 seconds). Then, every second imaging timing TR1 matches the display timing TF1. Note that, in the following, for convenience of description, of the imaging timings TR1, imaging timing TR1 matching the display timings TF1 will be described as imaging timing TR11 ((b) of FIG. 8), and imaging timing TR1 not matching the display timings TF1 will be described as imaging timing TR12 ((b) of FIG. 8).

Furthermore, the control unit 94 controls the light source device 3 as described below.

Specifically, as illustrated in (i) of FIG. 8, the control unit 94 causes the visible light source 31 to operate with every second imaging timing TR1 of a plurality of the imaging timings TR1, and causes the visible light source 31 to emit pulsed normal light. In other words, the visible light source 31 emits normal light in a time-division manner. In the present first embodiment, the control unit 94 causes the visible light source 31 to emit pulsed normal light with the imaging timing TR12 of the plurality of the imaging timings TR1.

Furthermore, as illustrated in (j) of FIG. 8, the control unit 94 causes the first to third excitation light sources 32 to 34 to operate, and causes the first to third excitation light sources 32 to 34 to simultaneously and continuously emit the blue excitation light, the red excitation light, and the infrared excitation light at all times.

Therefore, when all the horizontal lines in the effective pixel region of the imaging element 528 are exposed to the normal light, the normal-light image is generated in the imaging element 528. Meanwhile, when all the horizontal lines in the effective pixel region of the imaging element 528 are not exposed to the normal light, the fluorescence image is generated in the imaging element 528.

Note that, in FIG. 8, "WLI1" is described in a square indicating a normal-light image of the first frame, and "WLI2" to "WLI4" are described respectively in squares indicating normal-light images of the second to fourth frames. In addition, in FIG. 8, "FI1" is described in a square indicating a fluorescence image of the first frame, and "FI2" to "FI4" are described respectively in squares indicating fluorescence images of the second to fourth frames.

In addition, the memory controller 931 writes the captured image transmitted from the camera head 5 in the memory 92 as described below.

Specifically, the memory controller 931 starts writing a captured image to the memory 92 with imaging timing TR1, for the captured image transmitted from the camera head 5, and finishes the writing of the captured image with the next imaging timing TR1.

For example, as illustrated in (d) of FIG. 8, for the normal-light image of the first frame, the memory controller 931 starts writing to the bank 1 in the memory 92 with the imaging timing TR12, and finishes the writing to the bank 1 with the next imaging timing TR11.

Furthermore, for example, as illustrated in (e) of FIG. 8, for the fluorescence image of the first frame, the memory controller 931 starts writing to the bank 2 in the memory 92 with the imaging timing TR11 at which the writing of the normal-light image of the first frame to the bank 1 is finished, and finishes the writing to the bank 2 with the next imaging timing TR12.

Furthermore, for example, as illustrated in (f) of FIG. 8, for a normal-light image of the second frame, the memory controller 931 starts writing to the bank 3 in the memory 92 with the imaging timing TR12 at which the writing of the fluorescence image of the first frame to the bank 2 is finished, and finishes the writing to the bank 3 with the next imaging timing TR11.

Furthermore, for example, as illustrated in (g) of FIG. 8, for the fluorescent image of the second frame, the memory controller 931 starts writing to the bank 4 in the memory 92 with the imaging timing TR11 at which the writing to the bank 3 of the normal-light image of the second frame is finished, and finishes the writing to the bank 4 with the next imaging timing TR12.

Furthermore, for example, as illustrated in (d) of FIG. 8, for the normal-light image of the third frame, the memory controller 931 starts writing to the bank 1 in the memory 92 (change of writing from the normal-light image of the first frame) with the imaging timing TR12 at which the writing of the fluorescence image of the second frame to the bank 4 is finished, and finishes the writing to the bank 1 with the next imaging timing TR11.

Furthermore, for example, as illustrated in (e) of FIG. 8, for the fluorescence image of the third frame, the memory controller 931 starts writing to the bank 2 in the memory 92 (change of writing from the fluorescence image of the first frame) with the imaging timing TR11 at which the writing of the normal-light image of the third frame to the bank 1 is finished, and finishes the writing to the bank 2 with the next imaging timing TR12.

Furthermore, for example, as illustrated in (f) of FIG. 8, for the normal-light image of the fourth frame, the memory controller 931 starts writing to the bank 3 in the memory 92 (change of writing from the normal-light image of the second frame) with the imaging timing TR12 at which the writing of the fluorescence image of the third frame to the bank 2 is finished, and finishes the writing to the bank 3 with the next imaging timing TR11.

Furthermore, for example, as illustrated in (g) of FIG. 8, for the fluorescence image of the fourth frame, the memory controller 931 starts writing to the bank 4 in the memory 92 (change of writing from the fluorescence image of the second frame) with the imaging timing TR11 at which the writing of the normal-light image of the fourth frame to the bank 3 is finished, and finishes the writing to the bank 4 with the next imaging timing TR12.

In addition, the observation image generation unit 93 causes the display device 7 to display the captured image (superimposed image) as described below, under the control of the control unit 94.

Specifically, the memory controller 931 sequentially reads the captured images from the memory 92 with the display timings TF1. Furthermore, the image processing unit 932 performs image processing on the read captured images. Furthermore, the superimposed image generation unit 933 performs superimposition processing of generating the superimposed image by superimposing the normal-light image and the fluorescence image both of which have been subjected to the image processing in the image processing unit 932. Then, the display controller 934 generates the video signal for displaying the superimposed image generated in the superimposed image generation unit 933, and outputs the video signal to the display device 7. Therefore, one frame of the superimposed image is sequentially displayed on the display device 7 according to the display timing TF1.

For example, at the display timing TF1 at which the writing of the normal-light image of the first frame to the bank 1 in the memory 92 is finished, the fluorescence image is not yet written to the memory 92, and therefore, the normal-light image of the first frame is displayed on the display device 7 as the superimposed image, until the next display timing TF1, as illustrated in (c) of FIG. 8.

Furthermore, for example, at the display timing TF1 at which display of the normal-light image of the first frame is finished, (display timing TF1 at which the writing of the normal-light image of the second frame to the bank 3 in the memory 92 is finished), the superimposed image of the normal-light image of the second frame and the fluorescence image of the first frame is displayed on the display device 7 until the next display timing TF1, as illustrated in (c) of FIG. 8.

Furthermore, for example, at the display timing TF1 at which display of the superimposed image of the normal-light image of the second frame and the fluorescence image of the first frame is finished (display timing TF1 at which the writing of the normal-light image of the third frame to the bank 1 in the memory 92 is finished), the superimposed image of the normal-light image of the third frame and the fluorescence image of the second frame is displayed on the display device 7 until the next display timing TF1, as illustrated in (c) of FIG. 8.

Furthermore, for example, at the display timing TF1 at which display of the superimposed image of the normal-light image of the third frame and the fluorescence image of the second frame is finished (display timing TF1 at which the writing of the normal-light image of the fourth frame to the bank 3 in the memory 92 is finished), the superimposed image of the normal-light image of the fourth frame and the fluorescence image of the third frame is displayed on the display device 7 until the next display timing TF1, as illustrated in (c) of FIG. 8.

According to the present first embodiment described above, the following effects are obtained.

The light source device 3 according to the present first embodiment includes the visible light source 31 that emits normal light, and the first to third excitation light sources 32 to 34 that emit the blue excitation light, the red excitation light, and the infrared excitation light, respectively, corresponding to a plurality of kinds of drugs.

Therefore, according to the light source device 3 of the present first embodiment, fluorescent observation corresponding to the plurality of kinds of drugs can be performed, with improved convenience.

In addition, in the light source device 3 according to the present first embodiment, the visible light source 31 emits normal light in a time division manner. Meanwhile, the first to third excitation light sources 32 to 34 simultaneously and continuously emit the blue excitation light, the red excitation light, and the infrared excitation light.

Therefore, the exposure time to each of the blue fluorescence, the red fluorescence, and the infrared fluorescence can be increased, and a bright fluorescence image can be generated.

In addition, in the light source device 3 according to the present first embodiment, the arrangement order of the first to third excitation light sources 32 to 34 relative to the connector CN3 corresponds to the order of magnitude of the peak wavelengths of the excitation light emitted from the first to third excitation light sources 32 to 34. More specifically, the larger peak wavelengths the emitted excitation light of the first to third excitation light sources 32 to 34 have, the closer the first to third excitation light sources 32 to 34 are arranged to the connector CN3.

This configuration makes it possible to facilitate design of the first to third dichroic mirrors 35 to 37.

Furthermore, in the medical observation system 1 according to the present first embodiment, a difference in time between timing to finish generation of the normal-light image by the camera head 5 (imaging timing TR11 at which writing of the normal-light image to the memory 92 is finished) and timing to start display of the captured image on the display device 7 (display timing TF1) is smaller than a difference in time between timing to finish generation of the fluorescence image by the camera head 5 (imaging timing TR12 at which writing of the fluorescence image to the memory 92 is finished) and the timing to start display (the display timing TF1).

Therefore, it is possible to reduce a time lag from capturing the normal-light image to displaying the normal-light image

### (First modification)

Next, a first modification of the first embodiment described above will be described.

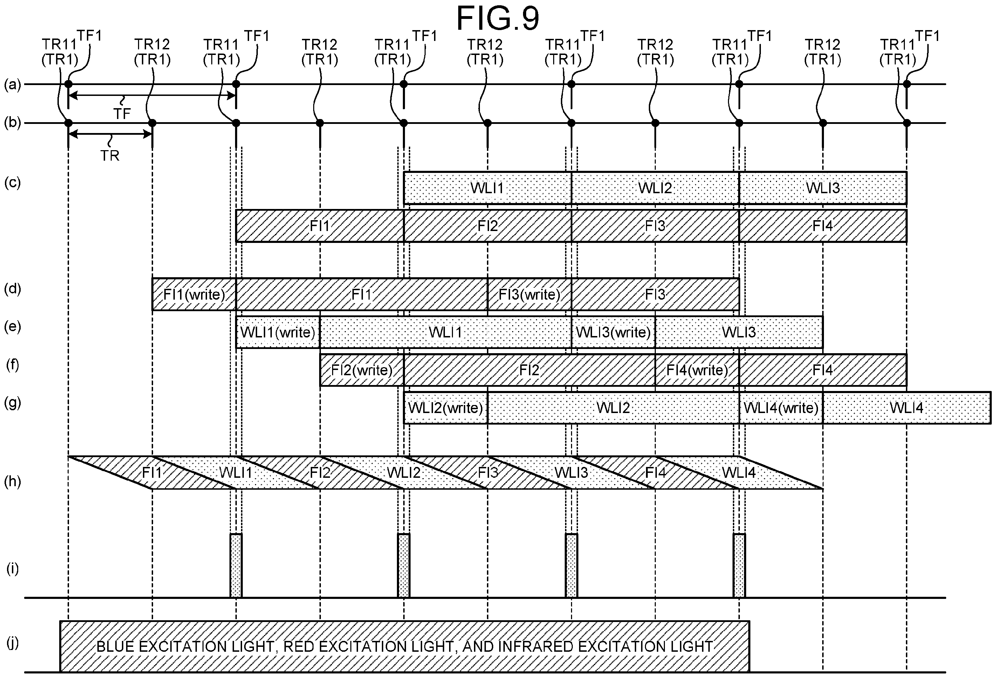

FIG. 9 is a diagram illustrating the first modification of the first embodiment. Specifically, FIG. 9 is a diagram corresponds to FIG. 8.

In the first embodiment described above, the control unit 94 has caused the visible light source 31 to emit pulsed normal light with the imaging timing TR12 of the plurality of the imaging timings TR1, but the present first modification is not limited thereto. For example, as illustrated in (i) of FIG. 9, the control unit 94 may cause the visible light source 31 to emit pulsed normal light with the imaging timing TR11 of the plurality of the imaging timings TR1.

Therefore, a difference in time between timing to finish generation of the fluorescence image by the camera head 5 (imaging timing TR11 at which writing of the fluorescence image to the memory 92 is finished) and timing to start display of the captured image on the display device 7 (display timing TF1) is smaller than a difference in time between timing to finish generation of the normal-light image by the camera head 5 (imaging timing TR12 at which writing of the normal-light image to the memory 92 is finished) and the timing to start display (the display timing TF1).

Therefore, according to the present first modification, it is possible to reduce a time lag from capturing the fluorescence image to displaying the fluorescence image.

### (Second embodiment)

Next, the present second embodiment will be described.

In the following description, configurations similar to those of the first embodiment described above are denoted by the same reference numerals, and detailed description thereof is omitted or simplified.

The present second embodiment is different from the first embodiment in operations of the control device 9. Hereinafter, the operations of the control device 9 according to the present second embodiment will be described.

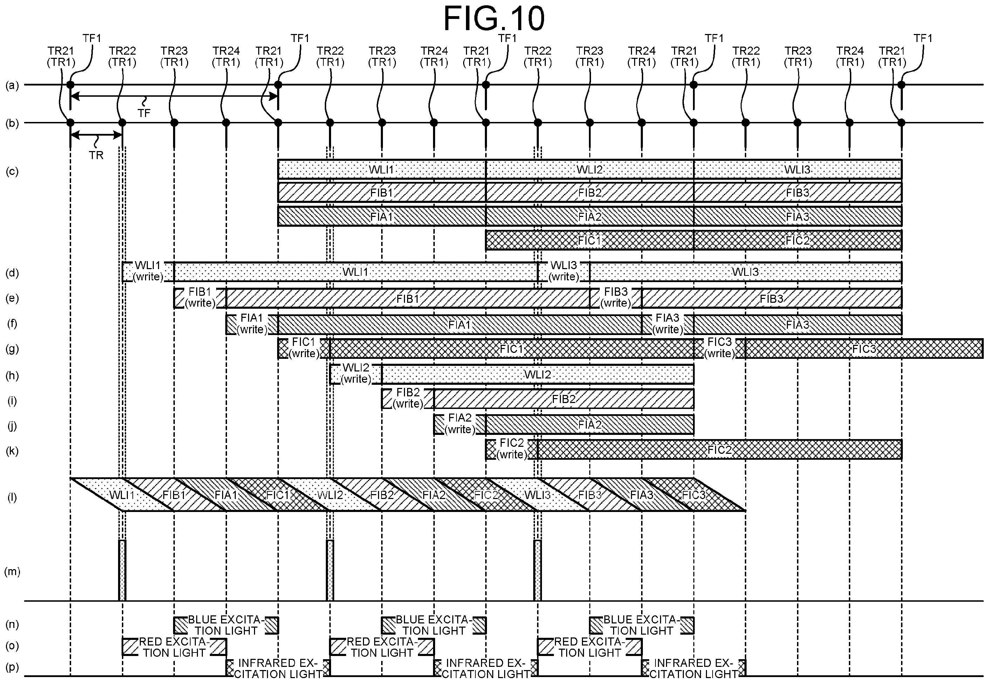

FIG. 10 is a diagram illustrating operations of the control device 9 according to the second embodiment. Specifically, a diagram of (a) of FIG. 10 corresponds to (a) of FIG. 8 and illustrates timing at which the control device 9 outputs the video signal according to a captured image (superimposed image) of one frame to the display device 7 to cause the display device 7 to display the captured image. A diagram of (b) of FIG. 10 corresponds to (b) of FIG. 8 and illustrates timing at which the imaging element 528 is caused to capture an image. A diagram of (c) of FIG. 10 corresponds to (c) of FIG. 8 and illustrates a captured image (superimposed image) output (displayed) on the display device 7. A diagram of (d) of FIG. 10 illustrates a captured image stored in the bank 1 in the memory 92. A diagram of (e) of FIG. 10 illustrates a captured image stored in the bank 2 in the memory 92. A diagram of (f) of FIG. 10 illustrates a captured image stored in the bank 3 in the memory 92. A diagram of (g) of FIG. 10 illustrates a captured image stored in the bank 4 in the memory 92. A diagram of (h) of FIG. 10 illustrates a captured image stored in a bank 5 in the memory 92. A diagram of (i) of FIG. 10 illustrates a captured image stored in a bank 6 in the memory 92. A diagram of (j) of FIG. 10 illustrates a captured image stored in a bank 7 in the memory 92. A diagram of (k) of FIG. 10 illustrates a captured image stored in a bank 8 in the memory 92. A diagram of (l) of FIG. 10 corresponds to (h) of FIG. 8 and illustrates exposure timing of the imaging element 528. A diagram of (m) of FIG. 10 corresponds to (i) of FIG. 8 and illustrates lighting timing of the visible light source 31. A diagram of (n) of FIG. 10 illustrates lighting timing of the first excitation light source 32. A diagram of (o) of FIG. 10 illustrates lighting timing of the second excitation light source 33. A diagram of (p) of FIG. 10 illustrates lighting timing of the third excitation light source 34.

In the present second embodiment, as illustrated in (a) of FIG. 10 and (b) of FIG. 10, the imaging period TR is set to 1/4 of the frame period TF (e.g., the frame period TF: 1/60 seconds and the imaging period TR: 1/240 seconds). Then, every fourth imaging timing TR1 matches the display timing TF1. Note that, in the following, for convenience of description, of the imaging timings TR1, imaging timing TR1 matching the display timing TF1 will be described as imaging timing TR21 ((b) of FIG. 10), imaging timing TR1 subsequent to the imaging timing TR21 will be described as imaging timing TR22 ((b) of FIG. 10), imaging timing TR1 subsequent to the imaging timing TR22 will be described as imaging timing TR23 ((b) of FIG. 10), and imaging timing TR1 subsequent to the imaging timing TR23 will be described as imaging timing TR24 ((b) of FIG. 10).

Then, as in the first embodiment described above, the control unit 94 performs exposure control using so-called rolling shutter to cause the imaging element 528 to start exposure with imaging timing TR1 sequentially from the uppermost horizontal line to the lowermost horizontal line and to read sequentially from the uppermost horizontal line to the lowermost horizontal line with the next imaging timing TR1 after a predetermined period (so-called shutter speed) has elapsed from the start of the exposure.

Furthermore, the control unit 94 controls the light source device 3 as described below.

Specifically, as illustrated in (m) of FIG. 10, the control unit 94 causes the visible light source 31 to operate with every fourth imaging timing TR1 of a plurality of the imaging timings TR1, and causes the visible light source 31 to emit pulsed normal light. In other words, the visible light source 31 emits normal light in a time-division manner. In the present second embodiment, the control unit 94 causes the visible light source 31 to emit pulsed normal light with the imaging timing TR22 of the plurality of the imaging timings TR1.

In addition, as illustrated in (n) of FIG. 10 to (p) of FIG. 10, the control unit 94 causes the first to third excitation light sources 32 to 34 to sequentially operate during an interval of emission of the pulsed normal light, for sequential emission from the first to third excitation light sources 32 to 34 with emission timing shifted. In the present second embodiment, the control unit 94 causes the second excitation light source 33 to emit the red excitation light between the imaging timing TR22 at which the pulsed normal light is caused to emit and the imaging timing TR24, causes the first excitation light source 32 to emit the blue excitation light between the imaging timing TR23 and the imaging timing TR21, and causes the third excitation light source 34 to emit the infrared excitation light between the imaging timing TR24 and the imaging timing TR22. In other words, to prevent continuous emission from the second and third excitation light sources 33 and 34 causing fluorescence having wavelength bands closest to each other during an interval of emission of the pulsed normal light, the first to third excitation light sources 32 to 34 sequentially emit the blue excitation light, the red excitation light, and the infrared excitation light, with emission timing shifted.

Here, as in the first embodiment described above, the image processing unit 932 generates a captured image of one frame from the first to third captured images mainly obtained by imaging normal light. In the following description, as in the first embodiment described above, the first to third captured images mainly obtained by imaging the normal light are collectively described as a normal-light image. Furthermore, the image processing unit 932 generates a captured image of one frame from the first to third captured images mainly obtained by imaging blue fluorescence. In the following description, for convenience of description, the first to third captured images mainly obtained by imaging the blue fluorescence are collectively described as a blue fluorescence image. Furthermore, the image processing unit 932 generates a captured image of one frame from the first to third captured images mainly obtained by imaging red fluorescence. In the following description, for convenience of description, the first to third captured images mainly obtained by imaging the red fluorescence will be collectively described as a red fluorescence image. Furthermore, the image processing unit 932 generates a captured image of one frame from the first to third captured images mainly obtained by imaging infrared fluorescence. In the following description, for convenience of description, the first to third captured images mainly obtained by imaging the infrared fluorescence are collectively described as an infrared fluorescence image.

Then, when all the horizontal lines in the effective pixel region of the imaging element 528 are exposed to the normal light, the normal-light image is generated in the imaging element 528. Furthermore, when all the horizontal lines in the effective pixel region of the imaging element 528 are exposed to the blue fluorescence, the blue fluorescence image according to the blue fluorescence is generated in the imaging element 528. Furthermore, when all the horizontal lines in the effective pixel region of the imaging element 528 are exposed to the red fluorescence, the red fluorescence image according to the red fluorescence is generated in the imaging element 528. Furthermore, when all the horizontal lines in the effective pixel region of the imaging element 528 are exposed to the infrared fluorescence, the infrared fluorescence image according to the infrared fluorescence is generated in the imaging element 528.

Note that, in FIG. 10, "WLI1" is described in a square indicating a normal-light image of the first frame, and "WLI2" and "WLI3" are described respectively in squares indicating normal-light images of the second and third frames. In addition, in FIG. 10, "FIA1" is described in a square indicating a blue fluorescence image of the first frame, and "FIA2" and "FIA3" are described respectively in the squares indicating blue fluorescence images of the second and third frames. Furthermore, in FIG. 10, "FIB1" is described in a square indicating a red fluorescence image of the first frame, and "FIB2" and "FIB3" are described respectively in squares indicating red fluorescence images of the second and third frames. In addition, in FIG. 10, "FIC1" is described in a square indicating an infrared fluorescence image of the first frame, and "FIC2" and "FIC3" are described respectively in squares indicating infrared fluorescence images of the second and third frames.

In addition, as in the first embodiment described above, the memory controller 931 starts writing a captured image to the memory 92 with imaging timing TR1, for the captured image transmitted from the camera head 5, and finishes the writing of the captured image with the next imaging timing TR1.

For example, as illustrated in (d) of FIG. 10, for the normal-light image of the first frame, the memory controller 931 starts writing to the bank 1 in the memory 92 with the imaging timing TR22, and finishes the writing to the bank 1 with the next imaging timing TR23.

Furthermore, for example, as illustrated in (e) of FIG. 10, for the red fluorescence image of the first frame, the memory controller 931 starts writing to the bank 2 in the memory 92 with the imaging timing TR23 at which the writing of the normal-light image of the first frame to the bank 1 is finished, and finishes the writing to the bank 2 with the next imaging timing TR24.

Furthermore, for example, as illustrated in (f) of FIG. 10, for the blue fluorescence image of the first frame, the memory controller 931 starts writing to the bank 3 in the memory 92 with the imaging timing TR24 at which the writing of the red fluorescence image of the first frame to the bank 2 is finished, and finishes the writing to the bank 3 at the next imaging timing TR21.

Furthermore, for example, as illustrated in (g) of FIG. 10, for the infrared fluorescence image of the first frame, the memory controller 931 starts writing to the bank 4 in the memory 92 with the imaging timing TR21 at which the writing of the blue fluorescence image of the first frame to the bank 3 is finished, and finishes the writing to the bank 4 with the next imaging timing TR22.

Furthermore, for example, as illustrated in (h) of FIG. 10, for the normal-light image of the second frame, the memory controller 931 starts writing to the bank 5 in the memory 92 with the imaging timing TR22 at which the writing of the infrared fluorescence image of the first frame to the bank 4 is finished, and finishes the writing to the bank 5 with the next imaging timing TR23.

Furthermore, for example, as illustrated in (i) of FIG. 10, for the red fluorescence image of the second frame, the memory controller 931 starts writing to the bank 6 in the memory 92 with the imaging timing TR23 at which the writing of the normal-light image of the second frame to the bank 5 is finished, and finishes the writing to the bank 6 with the next imaging timing TR24.

Furthermore, for example, as illustrated in (j) of FIG. 10, for the blue fluorescence image of the second frame, the memory controller 931 starts writing to the bank 7 in the memory 92 with the imaging timing TR24 at which the writing of the red fluorescence image of the second frame to the bank 6 is finished, and finishes the writing to the bank 7 with the next imaging timing TR21.

Furthermore, for example, as illustrated in (k) of FIG. 10, for the infrared fluorescence image of the second frame, the memory controller 931 starts writing to the bank 8 in the memory 92 with the imaging timing TR21 at which the writing of the blue fluorescence image of the second frame to the bank 7 is finished, and finishes the writing to the bank 8 with the next imaging timing TR22.

Furthermore, for example, as illustrated in (d) of FIG. 10, for the normal-light image of the third frame, the memory controller 931 starts writing to the bank 1 in the memory 92 (change of writing from the normal-light image of the first frame) with the imaging timing TR22 at which the writing of the infrared fluorescence image of the second frame to the bank 8 is finished, and finishes the writing to the bank 1 with the next imaging timing TR23.

Furthermore, for example, as illustrated in (e) of FIG. 10, for the red fluorescence image of the third frame, the memory controller 931 starts writing to the bank 2 in the memory 92 (change of writing from the red fluorescence image of the first frame) with the imaging timing TR23 at which the writing of the normal-light image of the third frame to the bank 1 is finished, and finishes the writing to the bank 2 with the next imaging timing TR24.

Furthermore, for example, as illustrated in (f) of FIG. 10, for the blue fluorescence image of the third frame, the memory controller 931 starts writing to the bank 3 in the memory 92 (change of writing from the blue fluorescence image of the first frame) with the imaging timing TR24 at which the writing of the red fluorescence image of the third frame to the bank 2 is finished, and finishes the writing to the bank 3 with the next imaging timing TR21.

Furthermore, for example, as illustrated in (g) of FIG. 10, for the infrared fluorescence image of the third frame, the memory controller 931 starts writing to the bank 4 in the memory 92 (change of writing from the infrared fluorescence image of the first frame) with the imaging timing TR21 at which the writing of the blue fluorescence image of the third frame to the bank 3 is finished, and finishes the writing to the bank 4 with the next imaging timing TR22.

In addition, as in the first embodiment described above, the observation image generation unit 93 causes the display device 7 to display the captured image as described below, under the control of the control unit 94.

Specifically, the memory controller 931 sequentially reads the captured images from the memory 92 with the display timings TF1. Furthermore, the image processing unit 932 performs image processing on the read captured images. Furthermore, the superimposed image generation unit 933 performs superimposition processing of generating the superimposed image by superimposing the normal-light image, the blue fluorescence image, the red fluorescence image, and the infrared fluorescence image all of which have been subjected to the image processing in the image processing unit 932. Then, the display controller 934 generates the video signal for displaying the superimposed image generated in the superimposed image generation unit 933, and outputs the video signal to the display device 7. Therefore, one frame of the superimposed image is sequentially displayed on the display device 7 according to the display timing TF1.

For example, at the display timing TF1 at which the writing of the blue fluorescence image of the first frame to the bank 3 in the memory 92 is finished, the infrared fluorescence image is not yet been written to the memory 92, and therefore, the superimposed image of the blue fluorescence image of the first frame, the normal-light image of the first frame, and the red fluorescence image of the first frame is displayed on the display device 7, until the next display timing TF1, as illustrated in (c) of FIG. 10.

Furthermore, for example, at the display timing TF1 at which the display of the superimposed image of the blue fluorescence image of the first frame, the normal-light image of the first frame, and the red fluorescence image of the first frame is finished ( display timing TF1 at which the writing of the blue fluorescence image of the second frame to the bank 7 in the memory 92 is finished), the superimposed image of the blue fluorescence image of the second frame, the normal-light image of the second frame, the red fluorescence image of the second frame, and the infrared fluorescence image of the first frame is displayed on the display device 7 until the next display timing TF1, as illustrated in (c) of FIG. 10.

Furthermore, for example, at the display timing TF1 at which the display of the superimposed image of the blue fluorescence image of the second frame, the normal-light image of the second frame, the red fluorescence image of the second frame, and the infrared fluorescence image of the first frame is finished (display timing TF1 at which the writing of the blue fluorescence image of third frame to the bank 3 in the memory 92 is finished), the superimposed image of the blue fluorescence image of the third frame, the normal-light image of the third frame, the red fluorescence image of the third frame, and the infrared fluorescence image of the second frame is displayed on the display device 7 until the next display timing TF1, as illustrated in (c) of FIG. 10.

According to the present second embodiment described above, the following effects are obtained, in addition to the effects similar to those of the first embodiment described above.

Incidentally, red fluorescence and infrared fluorescence have wavelength bands closer to each other, and the red fluorescence and the infrared fluorescence are mainly imaged by the third imaging element 527. Therefore, when the red fluorescence and the infrared fluorescence are simultaneously imaged by the third imaging element 527, a fluorescent region of the red fluorescence and a fluorescent region of the infrared fluorescence cannot be distinguished from each other in the captured image obtained by the imaging. In other words, even in the superimposed image finally generated, the fluorescent region of the red fluorescence and the fluorescent region of the infrared fluorescence cannot be distinguished from each other. Meanwhile, blue fluorescence is mainly captured by the first imaging element 525. Therefore, performing image processing on a captured image captured by the first imaging element 525 to set a fluorescent region of the blue fluorescence to a distinguishable color makes it possible to distinguish the fluorescent region of the blue fluorescence from other fluorescent regions of the red fluorescence and the infrared fluorescence, in the superimposed image finally generated.

In the light source device 3 according to the present second embodiment, the visible light source 31 emits normal light in a time division manner. Meanwhile, to prevent continuous emission from the second and third excitation light sources 33 and 34 causing fluorescence having wavelength bands closest to each other during an interval of emission of the pulsed normal light, the first to third excitation light sources 32 to 34 sequentially emit the blue excitation light, the red excitation light, and the infrared excitation light, with emission timing shifted.

Therefore, the red fluorescence and the infrared fluorescence having wavelength bands closer to each other can be shifted in imaging timing. In other words, it is possible to generate the red fluorescence image by imaging the red fluorescence, in a period in which no infrared excitation light is emitted and substantially no infrared fluorescence is generated. Similarly, it is possible to generate the infrared fluorescence image by imaging the infrared fluorescence in a period in which no red excitation light is emitted and substantially no red fluorescence is generated. Therefore, performing the image processing makes it possible to make the color of the fluorescent region included in the red fluorescence image different from the color of the fluorescent region included in the infrared fluorescence image to generate the superimposed image in which the red fluorescence and the infrared fluorescence can be identified.

### (Second modification)

Next, a second modification of the second embodiment described above will be described.

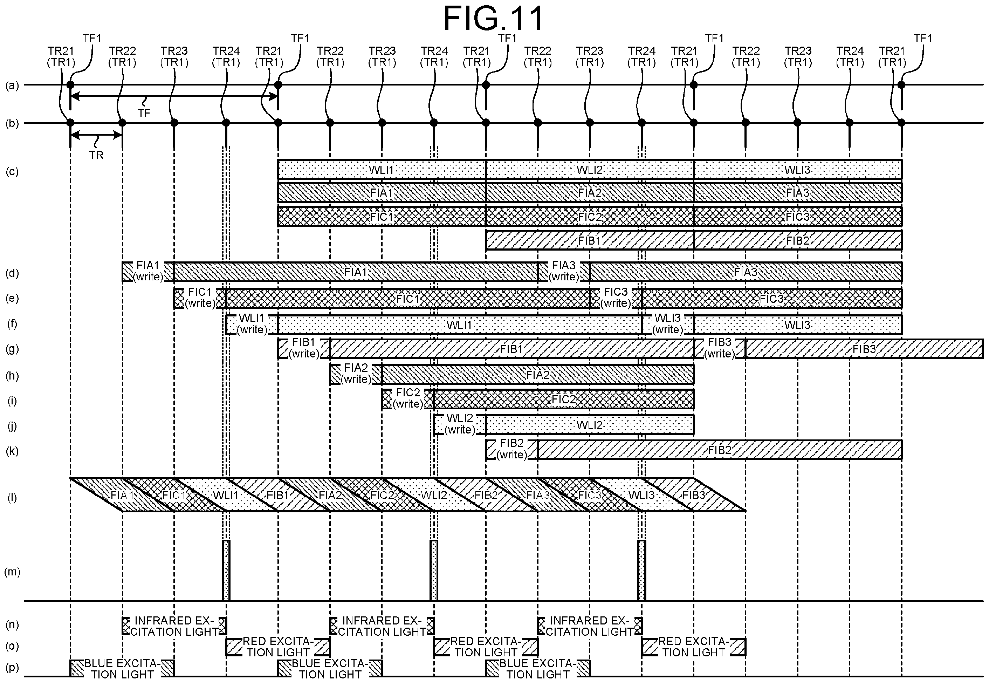

FIG. 11 is a diagram illustrating the second modification of the second embodiment. Specifically, FIG. 11 is a diagram corresponds to FIG. 10.

In the second embodiment described above, the control unit 94 has caused the visible light source 31 to emit pulsed normal light with the imaging timing TR22 of the plurality of the imaging timings TR1, but the present second modification is not limited thereto. For example, as illustrated in (m) of FIG. 11, the control unit 94 may cause the visible light source 31 to emit pulsed normal light with the imaging timing TR24 of the plurality of the imaging timings TR1. Note that, as illustrated in (n) of FIG. 11 to (p) of FIG. 11, the order of emitting the blue excitation light, the red excitation light, and the infrared excitation light during an interval of emission of the pulsed normal light is similar to that in the first embodiment described above.

Therefore, a difference in time between timing to finish generation of the fluorescence image by the camera head 5 (imaging timing TR21 at which writing of the normal-light image to the memory 92 is finished) and timing to start display of the captured image on the display device 7 (display timing TF1) is smaller than a difference in time between timing to finish generation of each of the fluorescence images, that is, the blue fluorescence image, the red fluorescence image, and the infrared fluorescence image by the camera head 5 (imaging timings TR22 to TR24 at which writing of the respective fluorescence images in the memory 92 is finished) and the timing to start display (display timing TF1).

Therefore, according to the present third modification, it is possible to reduce a time lag from capturing the normal-light image to displaying the normal-light image.

### (Other embodiments)

The embodiments for carrying out the present disclosure have been described above, but it should be understood that the present disclosure is not limited only to the first and second embodiments described above.

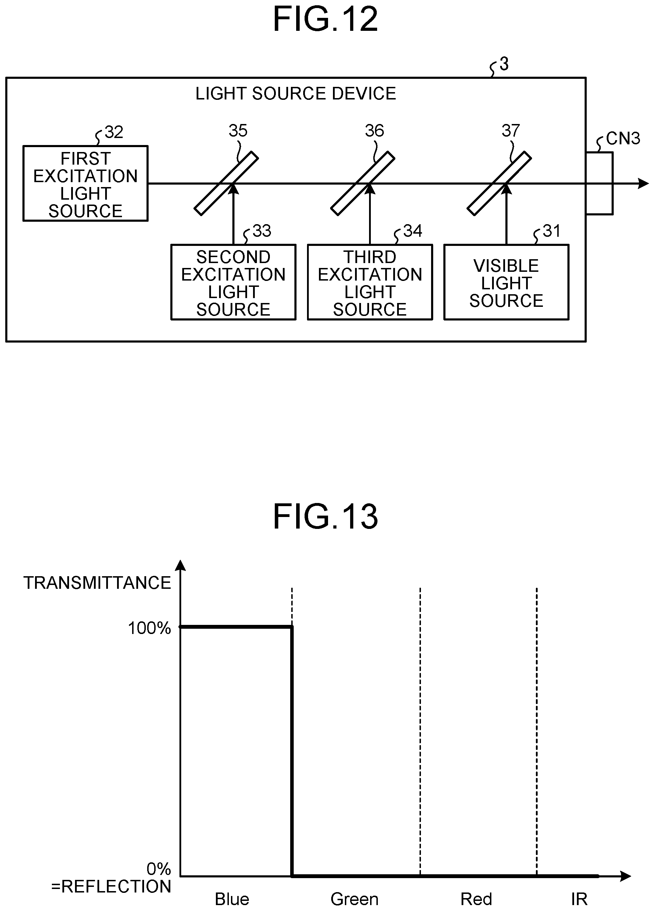

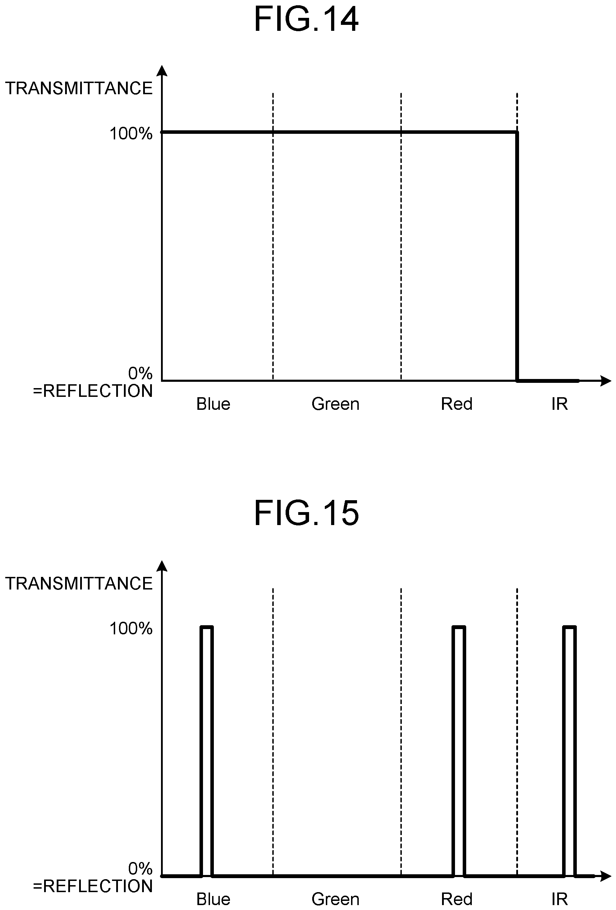

FIGS. 12 to 15 are each a diagram illustrating the third modification of the first and second embodiments. Specifically, FIG. 12 is a diagram illustrating a configuration of the light source device 3 according to the present third modification. FIG. 13 is a graph illustrating the characteristics of the first dichroic mirror 35 according to the present third modification. FIG. 14 is a graph illustrating the characteristics of the second dichroic mirror 36 according to the present third modification. FIG. 15 is a diagram illustrating the characteristics of the third dichroic mirror 37 according to the present third modification.

In the first and second embodiments described above, the light source device 3 according to the present third modification illustrated in FIG. 12 may be adopted.

As illustrated in FIG. 12, in the light source device 3 according to the present third modification, the arrangement positions of the visible light source 31 and the first to third excitation light sources 32 to 34 are changed from those in the first embodiment described above.

Specifically, as illustrated in FIG. 12, the larger peak wavelengths the emitted excitation light of the first to third excitation light sources 32 to 34 have, the closer the first to third excitation light sources 32 to 34 are arranged to the connector CN3. In Furthermore, the visible light source 31 is arranged at a position closer to the connector CN3 than the first to third excitation light sources 32 to 34.

Here, as illustrated in FIG. 13, the first dichroic mirror 35 according to the present third modification has characteristics of transmitting light in a blue wavelength band and reflecting light in the other wavelength bands. The first dichroic mirror 35 transmits the blue excitation light and reflects the red excitation light in the same direction as the travel direction of the blue excitation light.

Furthermore, as illustrated in FIG. 14, the second dichroic mirror 36 according to the present third modification has characteristics of reflecting light in an infrared wavelength band and transmitting light in other wavelength bands. Then, the second dichroic mirror 36 transmits the blue excitation light and the red excitation light, and reflects the infrared excitation light in the same direction as the travel directions of the blue excitation light and the red excitation light.

Furthermore, as illustrated in FIG. 15, the third dichroic mirror 37 according to the present third modification has characteristics of transmitting light in a wavelength band of the blue excitation light, a wavelength band of the red excitation light, and a wavelength band of the infrared excitation light, and reflecting light in the other wavelength bands. Then, the third dichroic mirror 37 transmits the blue excitation light, the red excitation light, and the infrared excitation light, and reflects the normal light in the same direction as those of the blue excitation light, the red excitation light, and the infrared excitation light.

Therefore, according to the present third modification, the dichroic mirrors having the characteristics as illustrated in FIGS. 13 to 15 are preferably designed as the first to third dichroic mirrors 35 to 37, further facilitating design of the first to third dichroic mirrors 35 to 37.

The excitation light (blue excitation light, red excitation light, and infrared excitation light) and the fluorescence (blue fluorescence, red fluorescence, and infrared fluorescence) generated by the excitation light, which have been described in the first and second embodiments and first to third modifications, are merely examples. In other words, the other wavelength bands may be adopted for the excitation light and the fluorescence. The number of the excitation light sources is not limited to three, and two, or four or more excitation light sources may be used.

In the above first and second embodiments and first to third modifications, the fluorescent observation has used all of the visible light, blue excitation light, red excitation light, and infrared excitation light that are emitted from the visible light source 31 and the first to third excitation light sources 32 to 34, but it is needless to say that the fluorescent observation can use the visible light and at least any of the blue excitation light, the red excitation light, and the infrared excitation light.

In the above first and second embodiments and first to third modifications, the imaging unit 52 with three CCDs including the dichroic prism 521 and the first to third imaging elements 525 to 527 has been adopted, but the present disclosure is not limited thereto, and an imaging unit with single CCD may be adopted as the imaging element.

In the above first and second embodiments and first to third modifications, the excitation light cut filters (the first to third excitation light cut filters 522 to 524) are provided between the first to third emergent surfaces 521B, 521G, and 521R and the first to third imaging elements 525 to 527, but the present disclosure is not limited thereto. For example, the excitation light cut filter may be provided on the incident side of the dichroic prism 521.

In the above first and second embodiments and first to third modifications, the superimposed image has been generated from the normal-light image and the fluorescence image (blue fluorescence image, red fluorescence image, and infrared fluorescence image) and displayed on the display device 7, but the present disclosure is not limited thereto. For example, the normal-light image and the fluorescence image (blue fluorescence image, red fluorescence image, and infrared fluorescence image) that are not superimposed but arranged side by side may be displayed on the display device 7.

In the above first and second embodiments and first to third modifications, the medical light source device according to the present disclosure has been mounted on the medical observation system 1 in which the insertion section 2 includes the rigid endoscope, but the present disclosure is not limited thereto. For example, the medical light source device according to the present disclosure may be mounted on a medical observation system in which the insertion section 2 includes a flexible endoscope. In addition, the medical light source device according to the present disclosure may be mounted on a medical observation system such as a surgical microscope (e.g., see JP 2016-42981 A) that observes an enlarged predetermined field of view in a living body or on a surface of the living body.

Additionally, the present disclosure may also be configured as below.
(1) A medical light source device including: a visible light source configured to emit normal light in a visible wavelength band; and a plurality of excitation light sources configured to emit a plurality of kinds of excitation light corresponding to a plurality of kinds of drugs each emitting fluorescence upon irradiation with the excitation light.
(2) The medical light source device according to (1), wherein the visible light source is configured to emit the normal light in a time division manner, and the plurality of excitation light sources are configured to simultaneously and continuously emit the plurality of kinds of excitation light.
(3) The medical light source device according to (1), wherein the visible light source is configured to emit the normal light in a time division manner, and the plurality of excitation light sources are configured to sequentially emit the plurality of kinds of excitation light with emission timing shifted during an interval of emission of the normal light from the visible light source in the time division manner.
(4) The medical light source device according to (3), wherein the plurality of excitation light sources include three or more excitation light sources and are configured to sequentially emit the plurality of kinds of excitation light with emission timing shifted in order that the excitation light sources whose wavelength bands of fluorescence to be generated are closest to each other do not continuously emit the plurality of kinds of excitation light, during the interval of emission of the normal light from the visible light source in the time division manner.
(5) The medical light source device according to any one of (1) to (4), further including a plurality of dichroic mirrors configured to cause the normal light emitted from the visible light source and the plurality of kinds of excitation light emitted from the plurality of excitation light sources to travel in an identical direction, wherein an arrangement order of the plurality of excitation light sources relative to an emission port at which the normal light and the plurality of kinds of excitation light are emitted from the medical light source device corresponds to an order of magnitude of peak wavelengths of the excitation light emitted from the plurality of excitation mirror light sources.
(6) The medical light source device according to (5), wherein the plurality of excitation light sources are arranged closer to the emission port as the excitation light to be emitted has a larger peak wavelength.
(7) The medical light source device according to (6), wherein the visible light source is arranged at a position closer to the emission port than the plurality of excitation light sources.
(8) A medical observation system including: a medical light source device including a visible light source configured to emit normal light in a visible wavelength band, and a plurality of excitation light sources configured to emit a plurality of kinds of excitation light corresponding to a plurality of kinds of drugs each emitting fluorescence upon irradiation with the excitation light; an imaging device configured to image the normal light emitted from the visible light source and reflected by a subject and fluorescence emitted from the plurality of kinds of drugs in the subject upon emission of the plurality of kinds of excitation light from the plurality of excitation mirror light sources; a display device configured to display a captured image captured by the imaging device; and a control device configured to control the medical light source device, the imaging device and the display device.
(9) The medical observation system according to (8), wherein the control device is configured to cause the imaging device to sequentially generate a normal-light image that is the captured image obtained by imaging the normal light and a fluorescence image that is the captured image obtained by imaging the fluorescence in a period shorter than a frame period in which the captured image of one frame is displayed on the display device, and a difference in time between timing to finish generation of the normal-light image by the imaging device and timing to start display of the captured image on the display device is smaller than a difference in time between timing to finish generation of the fluorescence image by the imaging device and the timing to start display.
(10) The medical observation system according to (8), wherein the control device is configured to cause the imaging device to sequentially generate a normal-light image that is the captured image obtained by imaging the normal light and a fluorescence image that is the captured image obtained by imaging the fluorescence in a period shorter than a frame period in which the captured image of one frame is displayed on the display device, and a difference in time between timing to finish generation of the fluorescence image by the imaging device and timing to start display of the captured image on the display device is smaller than a difference in time between timing to finish generation of the normal-light image by the imaging device and the timing to start display.

### Reference Signs List

- 1: MEDICAL OBSERVATION SYSTEM
- 2: INSERTION SECTION
- 3: LIGHT SOURCE DEVICE
- 4: LIGHT GUIDE
- 5: CAMERA HEAD
- 6: FIRST TRANSMISSION CABLE
- 7: DISPLAY DEVICE
- 8: SECOND TRANSMISSION CABLE
- 9: CONTROL DEVICE
- 10: THIRD TRANSMISSION CABLE
- 21: EYE PIECE
- 31: VISIBLE LIGHT SOURCE
- 32: FIRST EXCITATION LIGHT SOURCE
- 33: SECOND EXCITATION LIGHT SOURCE
- 34: THIRD EXCITATION LIGHT SOURCE
- 35: FIRST DICHROIC MIRROR
- 36: SECOND DICHROIC MIRROR
- 37: THIRD DICHROIC MIRROR
- 51: LENS UNIT
- 52: IMAGING UNIT
- 53: COMMUNICATION UNIT
- 91: COMMUNICATION UNIT
- 92: MEMORY
- 93: OBSERVATION IMAGE GENERATION UNIT
- 94: CONTROL UNIT
- 95: INPUT UNIT
- 96: OUTPUT UNIT
- 97: STORAGE UNIT
- 521: DICHROIC PRISM
- 521B: FIRST EMERGENT SURFACE
- 521G: SECOND EMERGENT SURFACE
- 521R: THIRD EMERGENT SURFACE
- 522: FIRST EXCITATION LIGHT CUT FILTER
- 523: SECOND EXCITATION LIGHT CUT FILTER
- 524: THIRD EXCITATION LIGHT CUT FILTER
- 525: FIRST IMAGING ELEMENT
- 526: SECOND IMAGING ELEMENT
- 527: THIRD IMAGING ELEMENT
- 528: IMAGING ELEMENT
- 931: MEMORY CONTROLLER
- 932: IMAGE PROCESSING UNIT
- 933: SUPERIMPOSED IMAGE GENERATION UNIT
- 934: DISPLAY CONTROLLER
- CN1 to CN3: CONNECTOR
- LB, LG, LR: LIGHT
- TF: FRAME PERIOD
- TF1: DISPLAY TIMING
- TR: IMAGING PERIOD
- TR1, TR11, TR12, TR21 to TR24: IMAGING TIMING

## Claims

1. A medical light source device comprising:
a visible light source configured to emit normal light in a visible wavelength band; and
a plurality of excitation light sources configured to emit a plurality of kinds of excitation light corresponding to a plurality of kinds of drugs each emitting fluorescence upon irradiation with the excitation light.

2. The medical light source device according to claim 1, wherein
the visible light source is configured to emit the normal light in a time division manner, and
the plurality of excitation light sources are configured to simultaneously and continuously emit the plurality of kinds of excitation light.

3. The medical light source device according to claim 1, wherein
the visible light source is configured to emit the normal light in a time division manner, and
the plurality of excitation light sources are configured to sequentially emit the plurality of kinds of excitation light with emission timing shifted during an interval of emission of the normal light from the visible light source in the time division manner.

4. The medical light source device according to claim 3, wherein
the plurality of excitation light sources include three or more excitation light sources and are configured to sequentially emit the plurality of kinds of excitation light with emission timing shifted in order that the excitation light sources whose wavelength bands of fluorescence to be generated are closest to each other do not continuously emit the plurality of kinds of excitation light, during the interval of emission of the normal light from the visible light source in the time division manner.

5. The medical light source device according to claim 1, further comprising
a plurality of dichroic mirrors configured to cause the normal light emitted from the visible light source and the plurality of kinds of excitation light emitted from the plurality of excitation light sources to travel in an identical direction, wherein
an arrangement order of the plurality of excitation light sources relative to an emission port at which the normal light and the plurality of kinds of excitation light are emitted from the medical light source device corresponds to an order of magnitude of peak wavelengths of the excitation light emitted from the plurality of excitation mirror light sources.

6. The medical light source device according to claim 5, wherein the plurality of excitation light sources are arranged closer to the emission port as the excitation light to be emitted has a larger peak wavelength.

7. The medical light source device according to claim 6, wherein the visible light source is arranged at a position closer to the emission port than the plurality of excitation light sources.

8. A medical observation system comprising:
a medical light source device including
a visible light source configured to emit normal light in a visible wavelength band, and
a plurality of excitation light sources configured to emit a plurality of kinds of excitation light corresponding to a plurality of kinds of drugs each emitting fluorescence upon irradiation with the excitation light;
an imaging device configured to image the normal light emitted from the visible light source and reflected by a subject and fluorescence emitted from the plurality of kinds of drugs in the subject upon emission of the plurality of kinds of excitation light from the plurality of excitation mirror light sources;
a display device configured to display a captured image captured by the imaging device; and
a control device configured to control the medical light source device, the imaging device and the display device.

9. The medical observation system according to claim 8, wherein
the control device is configured to cause the imaging device to sequentially generate a normal-light image that is the captured image obtained by imaging the normal light and a fluorescence image that is the captured image obtained by imaging the fluorescence in a period shorter than a frame period in which the captured image of one frame is displayed on the display device, and
a difference in time between timing to finish generation of the normal-light image by the imaging device and timing to start display of the captured image on the display device is smaller than a difference in time between timing to finish generation of the fluorescence image by the imaging device and the timing to start display.

10. The medical observation system according to claim 8, wherein
the control device is configured to cause the imaging device to sequentially generate a normal-light image that is the captured image obtained by imaging the normal light and a fluorescence image that is the captured image obtained by imaging the fluorescence in a period shorter than a frame period in which the captured image of one frame is displayed on the display device, and
a difference in time between timing to finish generation of the fluorescence image by the imaging device and timing to start display of the captured image on the display device is smaller than a difference in time between timing to finish generation of the normal-light image by the imaging device and the timing to start display.
